# EUROPEAN PATENT SPECIFICATION

(11) **EP 3 097 134 B1**
(45) Date of publication and mention of the grant of the patent: **19.07.2017**
(21) Application number: 15707538.3
(22) Date of filing: 21.01.2015
(51) Int. Cl.: C08G 63/46, A61K 47/34, C08F 299/04, C08G 63/664, C08G 63/91

(54) **BIODEGRADABLE HYDROGEL WITH CONTROLLED LIFETIME AND METHOD OF PREPARATION THEREOF**
BIOLOGISCH ABBAUBARES HYDROGEL MIT KONTROLLIERTER LEBENSDAUER UND VERFAHREN ZUR HERSTELLUNG DAVON
HYDROGEL BIODÉGRADABLE À DURÉE DE VIE CONTRÔLÉE ET SON PROCÉDÉ DE PRÉPARATION

(30) Priority: 23.01.2014 CZ 20140053; 23.01.2014 CZ 201429121 U
(43) Date of publication of application: 30.11.2016
(73) Proprietor: Vysoké ucení technické v Brne, 601 90 Brno (CZ)
(72) Inventor: VOJTOVA, Lucy, 664 57 Menin (CZ); MICHLOVSKA, Lenka, 690 02 Breclav (CZ); JANCAR, Josef, 623 00 Brno - Kohoutovice (CZ)
(74) Representative: Hartvichova, Katerina
(86) International application number: PCT/CZ2015/000006
(87) International publication number: WO 2015/110097

(56) References cited:
- WO-A1-00/38651
- WO-A1-2006/053936
- US-A- 5 410 016
- IVANA CHAMRADOVÁ ET AL: "Rheological properties of functionalised thermosensitive copolymers for injectable applications in medicine", CHEMICAL PAPERS, SP VERSITA, HEIDELBERG, vol. 66, no. 10, 23 June 2012 (2012-06-23) , pages 977-980, XP035083993, ISSN: 1336-9075, DOI: 10.2478/S11696-012-0210-Y

## Description

### Field of the Invention

The invention relates to a biodegradable hydrogel with controlled lifetime and a method of preparation thereof by chemical crosslinking without using any chemical crosslinker.

### Background Art

During the last two decades, biodegradable polymers and copolymers have met with increasing interest in biomedicine, particularly in the field of tissue engineering, cell therapies, wound treatment as well as drug carriers. The most frequently used polymers are linear aliphatic polyesters such as polyglycolide (PGA), polylactide (PLA) and their statistical copolymer of polylactide-co-polyglycolide (PLGA), whose ester bonds undergo homogenous hydrolysis.

Copolymer possesses the properties of individual homopolymers. The degradation rate of PLGA copolymer depends on its chemical structure and the homopolymers ratio of PLA and PGA. Hydrophobic PLGA copolymer is often modified by hydrophilic poly(ethylene glycol) (PEG). The most studied PLGA-PEG-PLGA triblock copolymer is nontoxic, biodegradable, biocompatible, resorbable and thermosensitive. Aqueous solution of PLGA-PEG-PLGA copolymer is liquid and behaves as a sol at room temperature and it changes to a stiff hydrogel at around the body temperature (Yu, L., Zhang, H., & Ding, J. (2010). Effects of precipitate agents on temperature-responsive sol-gel transitions of PLGA-PEG-PLGA copolymers in water. Colloid and Polymer Science, 288(10-11), pp. 1151-1159; Pratoomsoot, C., Tanioka, H., Hori, K., Kawasaki, S., Kinoshita, S., Tighe, P., Dua, H., Shakesheff, K., & Rose, F. (2008). A thermoreversible hydrogel as a biosynthetic bandage for corneal wound repair. Biomaterials, 29(3), pp. 272-281). After a specified time, it undergoes degradation by hydrolysis to nontoxic products and further by Krebs cycle to water and carbon dioxide. This copolymer is commercially known as injectable drug carrier ReGel® and it was used e.g. as insulin carrier for the treatment of Diabetes mellitus Type I. (Zentner, G., Rathi, R., Shih, C., McRea, J., Seo, M., Oh, H., Rhee, B., Mestecky, J., Moldoveanu, Z., Morgan, M., & Weitman, S. (2001). Biodegradable block copolymers for delivery of proteins and water-insoluble drugs. Journal of Controlled Release, 72(1-3), pp. 203-215). It is used in combination with paclitaxel drugs under the name OncoGel® for the treatment of cancer (Elstad, N., & Fowers, K. (2009). OncoGel (ReGel/paclitaxel) - Clinical applications for a novel paclitaxel delivery system. Advanced Drug Delivery Reviews, 61(10), pp. 785-794).

The use of the copolymers in medicine is limited due to the fact that the thermogelling sol-gel process is reversible and the copolymers have a low degree of functionality (they contain only hydroxyl functional groups). For this reason, much attention is paid to the modification of the functional groups and chemical (irreversible) crosslinking of these materials, which extend their possibility of application. Chemical crosslinking positively affects the rate and the mechanism of degradation of polymers. The resulting hydrogels are more stable, and therefore degrade more slowly (Zaldivar, D., Peniche, C., Gallardo, A., & Roman, J. (1993). Biocompatible hydrogels of controlled hydrophobicity from copolymers of N-vinyl-2-pyrrolidone and furfuryl methacrylate. Biomaterials, 14(14), pp. 1073-1079). These properties are particularly preferred for temporary orthopedic implants which gradually degrade during the healing and growth of human bones (Kharas, G., Kamenetsky, M., Simantirakis, J., Beinlich, K., Rizzo, A., Caywood, G., & Watson, K. (1997). Synthesis and characterization of fumarate-based polyesters for use in bioresorbable bone cement composites. Journal of Applied Polymer Science, 66(6), 1123-1137; Muggli, D., Burkoth, A., Keyser, S., Lee, H., & Anseth, K. (1998). Reaction Behavior of Biodegradable, Photo-Cross-Linkable Polyanhydrides. Macromolecules, 31(13), pp. 4120-4125).

Hydroxyl terminated poly(ε-caprolactone) (PCL), polylactide (PLA) and polyglycolide (PGA) were modified by maleic anhydride, fumaric acid, acrylate, methacrylic anhydride and triethoxy(3-isocyanatopropyl)silane in order to obtain functionalized polymers suitable for chemical crosslinking (Han, Y., Edelman, P., & Huang, S. (1988). Synthesis and Characterization of Crosslinked Polymers for Biomedical Composites. Journal of Macromolecular Science: Part A - Chemistry, 25(5-7), pp. 847-869; Turunen, M., Korhonen, H., Tuominen, J., & Seppälä, J. (2002). Synthesis, characterization and crosslinking of functional star-shaped poly(e-caprolactone). Polymer International, 51(1), pp. 92-100; Han, D., & Hubbell, J. (1997). Synthesis of Polymer Network Scaffolds from 1 -Lactide and Poly(ethylene glycol) and Their Interaction with Cells. Macromolecules, 30(20), pp. 6077-6083; Helminen, A., Korhonen, H., & Seppälä, J. (2001). Biodegradable crosslinked polymers based on triethoxysilane terminated polylactide oligomers. Polymer, 42(8), pp. 3345-3353; Tian, D., Dubois, P., & Jérôme, R. (1997). Biodegradable and biocompatible inorganic-organic hybrid materials. I. Synthesis and characterization. Journal of Polymer Science Part A: Polymer Chemistry, 35(11), 2295-2309; Korhonen, H., Helminen, A., & Seppälä, J. (2001). Synthesis of polylactides in the presence of coinitiators with different numbers of hydroxyl groups. Polymer, 42(18), pp. 7541-7549).

Use of itaconic anhydride for the modification of polymers has been reported only in the case of poly(ε-caprolactone) modification (Turunen, M., Korhonen, H., Tuominen, J., & Seppälä, J. (2002). Synthesis, characterization and crosslinking of functional star-shaped poly(ε-caprolactone). Polymer International, 51(1), pp. 92-100), poly(ε-caprolactone)/PEG (Ramos, M. (2002). Multi-component Hydrophilic-hydrophobic Systems from Itaconic Anhydride (Doctoral Dissertation Thesis). University of Connecticut, United States) and recently by the present invertors in an article where the conditions of functionalization by of PLGA-PEG-PLGA triblock copolymer were studied (Michlovská, L., Vojtová, L., Mravcová, L., Hermanová, S., Kučerík, J., & Jančář, J. (2010). Functionalization Conditions of PLGA-PEG-PLGA Copolymer with Itaconic Anhydride. Macromolecular Symposia, 295(1), pp. 119-124.). Crosslinked polyesters were formed by curing these functionalized polymers using thermoinitiators, redox systems or photoinitiators (Lang, M., & Chu, C. (2002-11-28). Functionalized multiarm poly(e-caprolactone)s: Synthesis, structure analysis, and network formation. Journal of Applied Polymer Science, 86(9), pp. 2296-2306; Lang, M., Wong, R., & Chu, C. (2002-04-15). Synthesis and structural analysis of functionalized poly (e-caprolactone)-based three-arm star polymers. Journal of Polymer Science Part A: Polymer Chemistry, 40(8), pp. 1127-1141.; Burdick, J., Philpott, L., & Anseth, K. (2001). Synthesis and characterization of tetrafunctional lactic acid oligomers: A potential in situ forming degradable orthopaedic biomaterial. Journal of Polymer Science Part A: Polymer Chemistry, 39(5), 683-692; Storey, R, Wiggins, J., Mauritz, K., & Puckett, A. (1993). Bioabsorbable composites. I: Fundamental design considerations using free radically crosslinkable matrices. Polymer Composites, 14(1), pp. 7-16.; Han, Y., Edelman, P., & Huang, S. (1988). Synthesis and Characterization of Crosslinked Polymers for Biomedical Composites. Journal of Macromolecular Science: Part A - Chemistry, 25(5-7), pp. 847-869; WO 03033563).

Modification of PLGA-PEG-PLGA copolymer using itaconic anhydride to give ITA/PLGA-PEG-PLGA/ITA has been recently published by the present inventors.

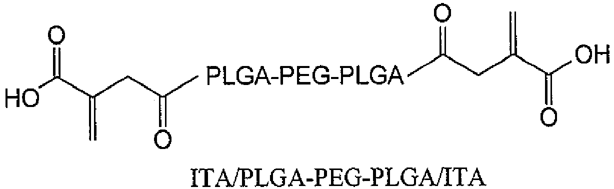

Itaconic anhydride is unsaturated cyclic anhydride and it can be obtained from renewable resources by both distillation of citric acid and pyrolysis of itaconic acid. Itaconic acid is produced mainly by fermentation of polysaccharides (e.g. molasses, glucose) using the bacteria *Aspergilus terreus* (US 6,171,831). It is generally known that itaconic anhydride decomposes to nontoxic degradation products under physiological conditions, initially hydrolyzing into itaconic acid in water. Adler et al. found out that the major degradation products acetate, lactate and carbon dioxide are formed by complete oxidation of itaconic acid using mitochondria obtained from guinea pig liver in the presence of Mg²⁺ (Adler, J., Wang, S., & Lardy. (1957). The Metabolism of Itaconic Acid by Liver Mitochondria. Journal of Biological Chemistry, 229(2), pp. 865-879). In a more recent publication by the present inventors (Chamradová I., Vojtová L., Michlovská L., Polácek P., Jancár J. (2012). Rheological properties of functionalised thermosensitive copolymers for injectable applications in medicine. Chemical Papers, 66(10), pp. 977-980) it was mentioned that the itaconic anhydride functionalized PLGA-PEG-PLGA copolymer can be chemically cross-linked by covalent bonding through the double bonds (e.g by photopolymerization) and/or physically, but no experimental details are given regarding the photopolymerization system.

Although patent application WO2006/053936 discloses a cross-linked copolymer produced by blue light photocuring in the presence of camphoroquinone and N,N-dimethylaminoethylmethacrylate, the copolymer is a methacrylated poly(lactic acid-itaconic acid-[epsilon]-caprolactone) resin, and the end product is used in coatings or composite materials, and not in the field of hydrogels for medical applications.

### Disclosure of the Invention

The object of the present invention is a biodegradable hydrogel with controlled lifetime obtainable by crosslinking α,ω-itaconyl terminated block copolymer [(polylactide-co-polyglycolide)-b-polyethyleneglycol-b-(polylactide-co-polyglycolide)] (ITA/PLGA-PEG-PLGA/ITA) using a blue light and catalyzed by a mixture of camphoroquinone and a tertiary amine without the presence of any crosslinking agent, wherein blue light is radiation with a wavelength in the range from 430 to 490 nm. In a preferred embodiment, the composition of the starting copolymer according to the invention can be described by formula I: 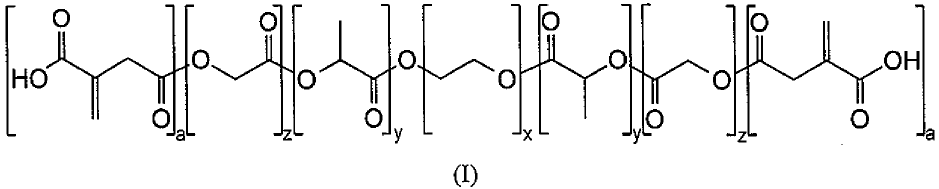 wherein the degrees of polymerization:
x (PEG) is in the range from 22 to 35;
2y (LA) independently is in the range from 19 to 41;
2z (GA) independently is in the range from 7 to 18;
and the degree of substitution:
2a (ITA) of itaconyl at the ends of the copolymer subsituting the original -OH groups is in the range from 0.35 to 2.

The tertiary amine is preferably a compound having the general formula R₃N⁺X-, wherein each R is independently selected from C1-C4 alkyl and X is a carboxylic acid anion, e.g. methacrylate, acrylate, benzoate. More preferably, the tertiary amine is selected from the group consisting of 2-(diethylamino)ethyl methacrylate (DEAEM) or ethyl 4-(dimethylamino) benzoate.

Aliphatic diketone 1,7,7-trimethyl-bicyclo[2.2.1]heptane-2,3-dione with a trivial name camphoroquinone (CQ) used as co-initiator has maximum efficiency at the wavelength of 468 nm. While camphoroquinone is partially oxidized to low reactive monoester and camphoric anhydride in the presence of oxygen, it is easily reduced to reactive α-hydroxyketones in an inert atmosphere, e.g. nitrogen. Therefore it is more preferable to carry out the crosslinking reaction in inert atmosphere.

Blue light is radiation with the wavelength in the range from 430 to 490 nm and it is commonly used e.g. in dental medicine for bleaching of teeth.

The object of the present invention is a method of preparation of the biodegradable hydrogel according to this invention. In the first step, [(polylactide-co-polyglycolide)-b-polyethyleneglycol-b-(polylactide-co-polyglycolide)] (PLGA-PEG-PLGA) block copolymer is modified by itaconic anhydride and subsequently chemically crosslinked by blue light at a temperature in the range from 23 to 130 °C under catalysis by a mixture of camphoroquinone and a tertiary amine without the presence of any crosslinking agent (Scheme 1).

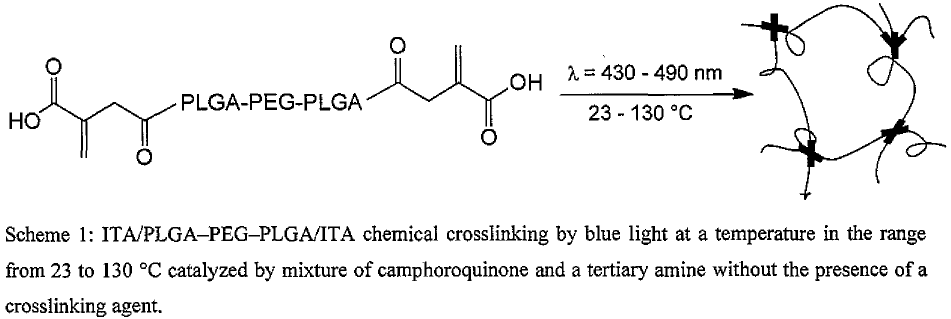

Organic solvents, e.g., acetone, tetrahydrofurane, dichloromethane, toluene, or their mixtures, may be used for complete dissolution of all components and absolute homogenization of the reaction mixture and they are evaporated after dosing the reaction mixture into molds. The solvents can be used directly in quality p.a. (per analysis) or after purification for example by distillation at atmospheric or reduced pressure in the presence of a hygroscopic desiccant (calcium hydride, calcium carbonate, metallic sodium/benzophenone, etc.).

### Brief description of drawings

Figure 1 shows swelling and degradation of crosslinked ITA/PLGA-PEG-PLGA/ITA samples in water at 20 °C.
Figure 2 shows crosslinked hydrogels a) ITA/PLGA-PEG-PLGA/ITA (with 37 mol% ITA) and b) ITA/PLGA-PEG-PLGA/ITA (with 63 mol% ITA) in swelled state on the 11^{th} day in water at laboratory temperature.
Figure 3 shows infrared spectra of the starting and the crosslinked copolymers.
Figure 4 shows magnification of the spectra of Fig. 3 in the region of 1560-1680 and 700-900 cm⁻¹.
Figure 5 shows decreasing amount of double bonds of the starting (original) copolymer and increasing amount of new RRC-CHR bonds of the crosslinked copolymer during crosslinking, determined by ATR-FTIR spectroscopy.
Figure 6 shows thermal stability of the starting (original) noncrosslinked and the crosslinked copolymers.
Figure 7 shows thermal stability of the starting (original) noncrosslinked and the crosslinked hydrogels determined by thermogravimetric analysis.

### Example of carrying out the Invention

ITA/PLGA-PEG-PLGA/ITA copolymers (used as macromonomers for crosslinking) with weight ratio of PLGA/PEG equal to 2.5 and molar ratio of LA/GA equal to 3.0 were prepared according to the publication MICHLOVSKÁ, Lenka, Lucy VOJTOVÁ, Ludmila MRAVCOVÁ, Soňa HERMANOVÁ, Jiří KUČERÍK and Josef JANČÁŘ. Functionalization Conditions of PLGA-PEG-PLGA Copolymer with Itaconic Anhydride. Macromolecular Symposia. 2010, 295 (1), p. 119-124). The amount of itaconic acid bonded on the ends of the polymer was 37 mol% and 63 mol% with the degree of substitution equal to 0.74 and 1.26 (for polymer with 37 and 63 mol%, respectively) - from the maximum value 2 corresponding to both ends of the polymer may be functionalized by ITA. The macromonomers were purified in phosphate buffer saline at pH 7.48 (PBS 7.4).

ITA binding to the ends of polymer was confirmed by proton nuclear magnetic resonance spectroscopy (¹H NMR). The amount of end-capped ITA was determined from integrals of characteristic proton intensities of itaconic acid double bonds (OC(CH2)CCH2COOH) at δ = 5.7 - 5.8 ppm and δ = 6.35 - 6.5 ppm and itaconic acid backbone (OCH2(C=O)) at δ = 3.40 - 3.44 ppm.

Preparation of the crosslinked hydrogel was carried out under nitrogen atmosphere. 0.2 g of ITA/PLGA-PEG-PLGA/ITA copolymer pre-dried in a desiccator for 30 minutes was placed on a thermoblock set up to 80 °C. 0.05 ml of solution of camphoroquinone in acetone with concentration 0.674 mol dm⁻³ and 0.003 ml of 2-(dimethylamino)ethyl methacrylate (DMAEM) used as catalyst were added to the sample.

The sample was dosed into a mold and chemically crosslinked by blue light. The crosslinking time was either 5 or 40 minutes for the first copolymer (37 mol% ITA) and 40 minutes for the second copolymer (63 mol% ITA). The samples were dried in vacuum oven at laboratory temperature for 8 hours. Dry xerogel was stored under nitrogen atmosphere in a fridge for further use and analysis.

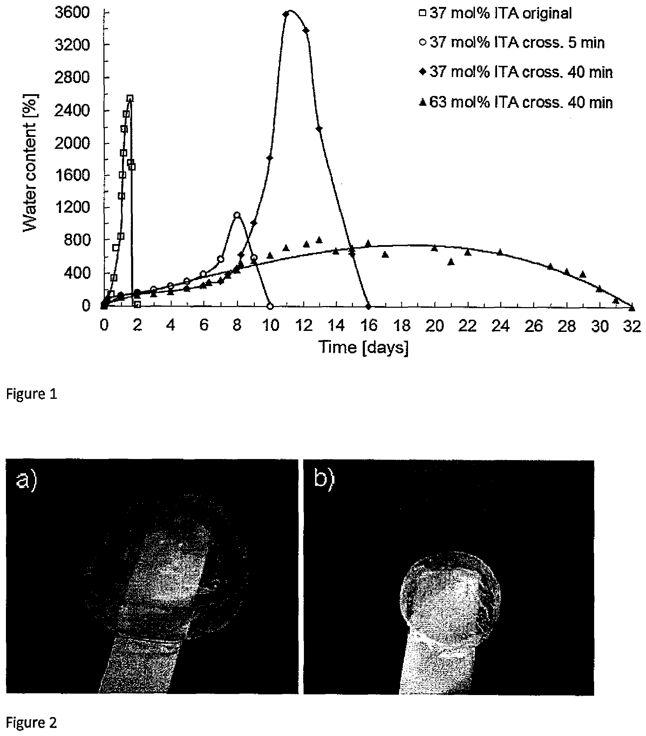

Hydrolytical stability and degradation of samples were measured in ultrapure water with pH equal to 6.7 at laboratory temperature. From the graph (Figure 1) it is apparent that the starting non-crosslinked copolymer with 37 mol% of ITA absorbed water from the beginning of the swelling very quickly up to 2547 % when it started degrade and it was completely dissolved after 48 hours. In contrast, in the copolymer crosslinked for 5 minutes, there occurred a gradual diffusion of water into the crosslinked polymer network during the first 7 days with slowly increasing water uptake up to 574 %. Water first hydrated hydrophilic groups of PEG and carboxyl end groups (primary bond water) and later on, part of the ester bonds started to hydrolyze. The density of the network decreased and the sample was able to absorb extra large amount of water up to 1103 % (secondary bond water). Subsequently, the network nodes were broken and hydrolysis of the rest of the PLGA ester bonds began together with the release of lactic and glycolic acid. For this reason, the dissolution of the sample was faster and the sample was completely dissolved after 10 days from the beginning of the swelling. If the same sample was crosslinked for a longer time (40 minutes), there was a significantly higher increase in the crosslinking density and the secondary binding of water in the sample started after the 9^{th} day to a maximum of 3581% (weight increased almost 37×). The sample started to dissolve after 11 days and it was wholly degraded on the 16^{th} day.

A different situation was observed when the ITA/PLGA-PEG-PLGA/ITA copolymer with 63 mol% of ITA was crosslinked by blue lamp for 40 minutes.The sample swelled in the same way as the crosslinked copolymer with 37 mol% ITA up to the 8^{th} day. After that there was only a slow increase of water content up to 813 %. In the period between the 13^{th} and 20^{th} day, there was a balance between diffusion and hydrolysis of the samples and then both degradation and dissolution. The sample was dissolved after 32 days. From this graph it is apparent that the period of hydrolytical stability (degradation time) can be affected both by the crosslinking time and the amount of bonded ITA.

ITA/PLGA-PEG-PLGA/ITA copolymer with 37 mol% ITA (a) and copolymer with 63 mol% ITA (b) in swelled state on the 11^{th} day of swelling is shown in Figure 2. The copolymer with 37 mol% ITA was able to absorb more water due to lower density of network and amount of network nodes.

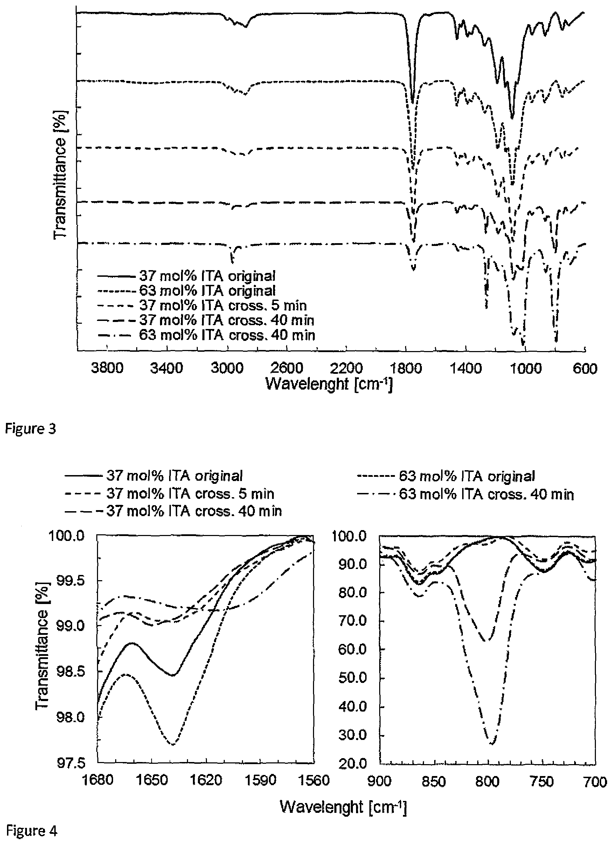

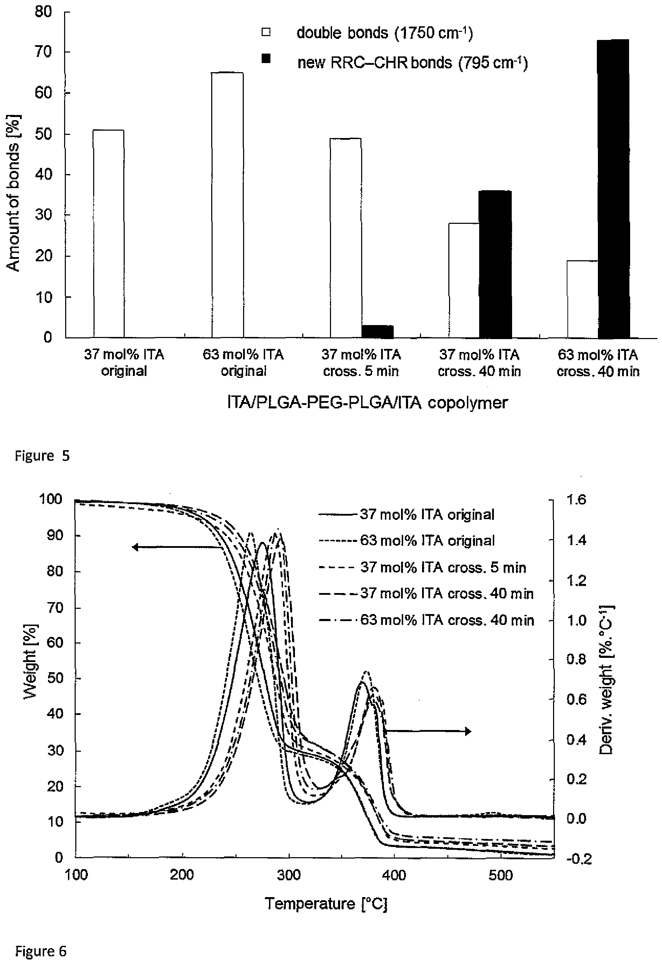

Figure 3 shows infrared spectra of original and crosslinked copolymers. The amount of bonded ITA and crosslinking time was observed as a decrease in the amount of double bonds at the wavelength of 1750 cm⁻¹ and an increase of the amount of new RRC-CHR bonds at 795 cm⁻¹ (Figure 4, Table 1). Original samples with 37 mol% and 63 mol% ITA contained 51 and 65 % of double bonds, respectively. There was only a 4% reduction of the double bonds and 3% increase of new bonds after crosslinking of copolymer with 37 mol% ITA for 5 minutes (Figure 5).

Table 1 shows molecular weights and polydispersity indexes of ITA/PLGA-PEG-PLGA/ITA copolymers.

**Table 1 Percentage distribution of individual bonds in the ATR-FTIR spectra.**

| ITA/PLGA-PEG-PLGA/ITA copolymer | Double bonds [%] | New bonds [%] |
|---|---|---|
| 37 mol% ITA original | 51 | 0 |
| 63 mol% ITA original | 65 | 0 |
| 37 mol% ITA cross. 5 min | 49 | 3 |
| 37 mol% ITA cross. 40 min | 28 | 36 |
| 63 mol% ITA cross. 40 min | 19 | 73 |

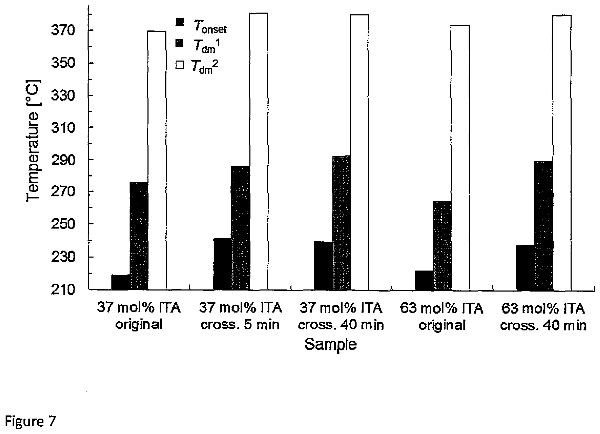

The crosslinked samples had a better thermal stability (Figure 6 and Table 2). The crosslinked samples started to degrade approx. from 239 °C, in comparison with the original (starting, noncrosslinked) copolymers, which degraded approx. at 220 °C (*T*ₒₙₛₑt) (Figure 7). For the copolymer with 63 mol% ITA, a noticeably increased temperature of the maximum rate of degradation of ester chains (T_{dm}¹) and a slight decomposition of ether chains (*T*_{dm}²) was observed, compared with the temperatures for the original copolymer being 264 to 290 °C and 373 to 380 °C, respectively. By calculation based on changes of the second peak at 344 °C, crosslinking conversion equal to 78.2 % was determined for samples crosslinked for 40 minutes with 63 mol% of ITA. Temperature at maximum degradation rate *T*_{dm}¹ increased from 276 to 286 and 293 °C for the copolymer with 37 mol% ITA crosslinked for 5 and 40 minutes and temperature of decomposition of ether bonds (*T*_{dm}²) from 369 to 381 and 380 °C.

**Table 2 Thermal stability of original non-crosslinked and crosslinked hydrogels determined by thermogravimetric analysis.**

| Temperature [°C] | ITA/PLGA-PEG-PLGA/ITA copolymer | | | | |
|---|---|---|---|---|---|
| | 37 mol% ITA original | 63 mol% ITA original | 37 mol% ITA cross. 5 min | 37 mol% ITA cross. 40 min | 63 mol% ITA cross. 40 min |
| Tₒₙₛₑₜ | 219 | 222 | 239 | 240 | 238 |
| T_{dm}¹ | 276 | 264 | 286 | 293 | 290 |
| T_{dm}² | 369 | 373 | 381 | 380 | 380 |

### Industrial Applicability

Biodegradable hydrogels according to the invention will be useful in particular in medical applications, e.g. for wet wound coverings of burns or as carriers for cells in tissue engineering of bone and cartilage.

## Claims

1. A biodegradable hydrogel with controlled lifetime obtainable by crosslinking α,ω-itaconyl terminated [(polylactide-co-polyglycolide)-b-polyethyleneglycol-b-(polylactide-co-polyglycolide)] block copolymer having the formula I: 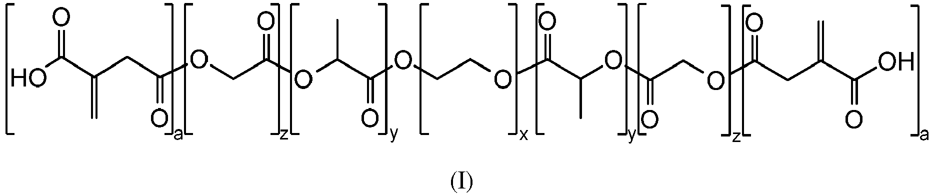 wherein degree of polymerization
x (PEG) is in the range from 22 to 35;
2y (LA) independently is in the range from 19 to 41;
2z (GA) independently is in the range from 7 to 18; and degree of substitution
2a (ITA substituting -OH groups at the ends of the starting copolymer) is in the range from 0.35 to 2
using a blue light and catalyzed by a mixture of camphoroquinone and a tertiary amine without the presence of any crosslinking agent,
wherein blue light is radiation with the wavelength in the range from 430 to 490 nm.

2. A method of preparation of biodegradable hydrogel according to claim 1, **characterized in that** in the first step the (polylactide-co-polyglycolide)-b-polyethylene glycol-b-(polylactide-co-polyglycolide) block copolymer is modified by itaconic anhydride, and subsequently the modified block copolymer is chemically crosslinked by blue light at a temperature in the range from 23 to 130 °C catalyzed by mixture of camphoroquinone and a tertiary amine without the presence of any crosslinking agent,
wherein blue light is radiation with the wavelength in the range from 430 to 490 nm.

3. The method according to claim 2, **characterized in that** the tertiary amine has a general formula R₃N⁺X⁻, wherein each R is independently selected from C1-C4 alkyl, and X is a carboxylic acid anion.

4. Method according to claim 3, **characterized in that** the tertiary amine is selected from the group consisting of 2-(diethylamino) ethyl methacrylate and ethyl 4-(dimethylamino)benzoate.

5. Method according to claim 2, **characterized in that** the crosslinking is carried out in the presence of an inert atmosphere.

6. Composition for the preparation of the hydrogel of claim 1, **characterized in that** in contains α,ω-itaconyl terminated [(polylactide-co-polyglycolide)-b-polyethyleneglycol-b-(polylactide-co-polyglycolide)] block copolymer, camphoroquinone and tertiary amine.

## Patentansprüche

1. Ein biologisch abbaubares Hydrogel mit kontrollierter Lebensdauer, erhältlich durch Vernetzung von α,ω-Itaconyl-terminiertem [(Polylactid-co-polyglykolid)-b-polyethylenglykol-b-(polylactid-co-polyglycolid)]-blockcopolymer mit der Formel I: 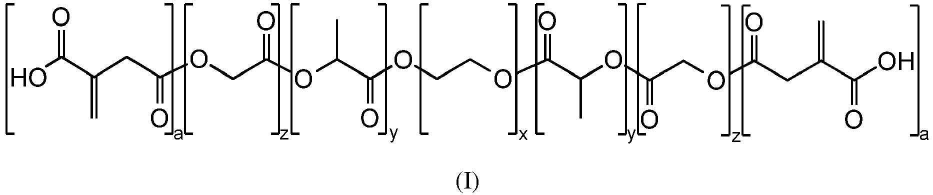 wobei der Polymerisationsgrad
x (PEG) im Bereich von 22 bis 35 liegt;
2y (LA) unabhängig im Bereich von 19 bis 41 liegt;
2z (GA) unabhängig im Bereich von 7 bis 18 liegt;
und Substitutionsgrad
2a (ITA-substituierte -OH-Gruppen an den Enden des Ausgangscopolymers) liegt im Bereich von 0,35 bis 2
mit einem blauen Licht und katalysiert durch eine Mischung aus Camphorchinon und einem tertiären Amin ohne die Anwesenheit eines Vernetzungsmittels,
wobei blaues Licht Strahlung mit der Wellenlänge im Bereich von 430 bis 490 nm ist.

2. Verfahren zur Herstellung eines biologisch abbaubaren Hydrogels nach Anspruch 1, **dadurch gekennzeichnet, daß** im ersten Schritt das (Polylactid-co-polyglykolid)-b-polyethylenglykol-b-(polylactid-co-polyglycolid)-blockcopolymer durch Itaconanhydrid modifiziert wird, und anschließend wird das modifizierte Blockcopolymer durch blaues Licht bei einer Temperatur im Bereich von 23 bis 130 °C, die durch eine Mischung aus Camphorchinon und einem tertiären Amin ohne die Anwesenheit eines Vernetzungsmittels katalysiert wird, chemisch vernetzt,
wobei blaues Licht Strahlung mit der Wellenlänge im Bereich von 430 bis 490 nm ist.

3. Verfahren nach Anspruch 2, **dadurch gekennzeichnet, dass** das tertiäre Amin die allgemeine Formel R₃N⁺X⁻ aufweist, wobei jedes R unabhängig ausgewählt ist aus C1-C4-Alkyl, und X ein Carbonsäureanion ist.

4. Verfahren nach Anspruch 3, **dadurch gekennzeichnet, dass** das tertiäre Amin ausgewählt ist aus der Gruppe bestehend aus 2-(Diethylamino)ethylmethacrylat und Ethyl-4-(dimethylamino)benzoat.

5. Verfahren nach Anspruch 2, **dadurch gekennzeichnet, dass** die Vernetzung in Gegenwart einer inerten Atmosphäre erfolgt.

6. Zusammensetzung zur Herstellung des Hydrogels nach Anspruch 1, **dadurch gekennzeichnet, daß** sie α,ω-Itaconyl-terminiertem [(Polylactid-co-polyglykolid)-b-polyethylenglykol-b-(polylactid-co-polyglycolid)]-blockcopolymer, Camphorchinon und tertiärem Amin enthält.

## Revendications

1. Un hydrogel biodégradable avec durée de vie contrôlée pouvant être obtenu par réticulation du copolymère séquencé [(polylactide-co-polyglycolide)-b-polyéthylèneglycol-b-(polylactide-co-polyglycolide)] à terminaison α,ω-itaconyle, le copolymère ayant la formule I: 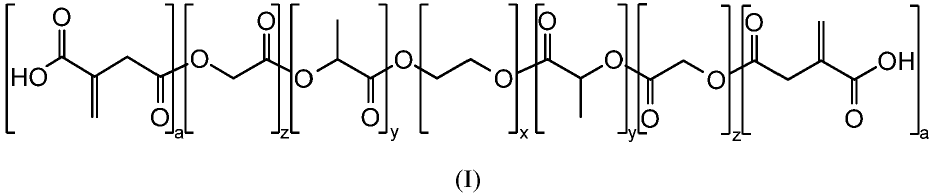 dans lequel le degré de polymérisation
x (PEG) se situe entre 22 et 35;
2y (LA) indépendamment dans la gamme de 19 à 41;
2z (GA) indépendamment dans la gamme de 7 à 18;
et le degré de substitution
2a (ITA substituant des groupes -OH aux extrémités du copolymère de départ) est compris entre 0,35 et 2
en utilisant une lumière bleue et catalysée par un mélange de camphoroquinone et d'une amine tertiaire sans présence d'agent de réticulation,
où la lumière bleue est un rayonnement avec la longueur d'onde entre 430 et 490 nm.

2. Procédé de préparation d'hydrogel biodégradable selon la revendication 1, **caractérisé en ce que** dans la première étape, le copolymère séquencé (polylactide-co-polyglycolide)-b-polyéthylèneglycol-b-(polylactide-co-polyglycolide) est modifié par itaconic anhydride et ensuite le copolymère séquencé modifié est chimiquement réticulé par de la lumière bleue à une température dans la gamme de 23 à 130 °C, catalysée par un mélange de camphoroquinone et d'une amine tertiaire sans présence d'agent de réticulation,
où la lumière bleue est un rayonnement avec la longueur d'onde entre 430 et 490 nm.

3. Procédé selon la revendication 2, **caractérisé en ce que** l'amine tertiaire a une formule générale R₃N⁺X⁻, dans laquelle chaque R est indépendamment choisi parmi alkyle en C1-C4, et X représente un anion d'acide carboxylique.

4. Procédé selon la revendication 3, **caractérisé en ce que** l'amine tertiaire est choisi dans le groupe consistant en le méthacrylate de 2-(diéthylamino)éthyle et le 4-(diméthylamino) benzoate d'éthyle.

5. Procédé selon la revendication 2, **caractérisé en ce que** la réticulation est réalisée en présence d'une atmosphère inerte.

6. Composition pour la préparation de l'hydrogel selon la revendication 1, **caractérisée en ce qu'**elle contient du copolymère séquencé [(polylactide-co-polyglycolide)-b-polyéthylèneglycol-b-(polylactide-co-polyglycolide)] à terminaison α,ω-itaconyle, la camphinoquinone et l'amine tertiaire.
